(19) Europäisches Patentamt
European Patent Office
Office européen des brevets

(11) **EP 3 261 511 B1**

(12) **EUROPÄISCHE PATENTSCHRIFT**

(45) Veröffentlichungstag und Bekanntmachung des Hinweises auf die Patenterteilung:
**20.01.2021   Patentblatt 2021/03**

(21) Anmeldenummer: **16702705.1**

(22) Anmeldetag: **02.02.2016**

(51) Int Cl.:
*A61B 1/00* (2006.01)          *G02B 23/24* (2006.01)
*G02B 25/00* (2006.01)          *G02B 27/42* (2006.01)

(86) Internationale Anmeldenummer:
**PCT/EP2016/052114**

(87) Internationale Veröffentlichungsnummer:
**WO 2016/134925 (01.09.2016 Gazette 2016/35)**

(54) **OKULAREINRICHTUNG FÜR EIN CHIRURGISCHES INSTRUMENT**

EYEPIECE DEVICE FOR A SURGICAL INSTRUMENT

SYSTÈME D'OCULAIRE POUR UN INSTRUMENT CHIRURGICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.02.2015   DE 102015203351**

(43) Veröffentlichungstag der Anmeldung:
**03.01.2018   Patentblatt 2018/01**

(73) Patentinhaber: **Olympus Winter&Ibe GmbH 22045 Hamburg (DE)**

(72) Erfinder: **KIEDROWSKI, Gregor 22397 Hamburg (DE)**

(74) Vertreter: **Seemann & Partner Patentanwälte mbB Raboisen 6 20095 Hamburg (DE)**

(56) Entgegenhaltungen:
WO-A1-2013/102476          CN-A- 102 004 309
US-A- 5 569 163               US-A1- 2001 036 019
US-A1- 2006 187 323

**Beschreibung**

Die Erfindung betrifft eine Okulareinrichtung für ein chirurgisches Instrument, insbesondere für ein Endoskop, wobei die Okulareinrichtung eine Okularfassung und zumindest eine optische Einheit, die in der Okularfassung aufgenommen ist, umfasst, wobei die optische Einheit zumindest ein erstes optisches Element und ein damit verbundenes zweites optisches Element umfasst, wobei das zweite optische Element aus einem zweiten Material mit anomaler Dispersion hergestellt ist und das erste und das zweite optische Element gemeinsam eine bezüglich chromatischer Aberration korrigierte optische Einheit bilden. Ferner betrifft die Erfindung ein Okular umfassend eine solche Okulareinrichtung sowie ein chirurgisches Instrument mit einem solchen Okular.

Chirurgische Instrumente, beispielsweise Endoskope, für minimalinvasive Eingriffe am menschlichen oder tierischen Körper sind allgemein bekannt. Bei einem Endoskop wird mit Hilfe einer Optik an einer distalen Spitze eines Endoskopschafts ein Operations- oder Untersuchungsfeld im Körperinneren betrachtet. Hierzu sind im Endoskopschaft mehrere optische Baugruppen angeordnet, mit denen Licht aus einem Körperhohlraum hinaus zu einem proximalen Ende des Endoskops geführt wird.

Am proximalen Ende des Endoskops, beispielsweise an einem Griff mit dem das Endoskop bedient wird, befindet sich vielfach ein Okulartrichter mit einem Okular. An dieser optischen Baugruppe tritt das an der distalen Spitze des Endoskops eintretende Licht wieder aus dem Endoskop aus. Das Okular dient teilweise zur direkten Beobachtung des Operationsfelds mit bloßem Auge. Vielfach ist jedoch vorgesehen, dass an das Okular ein Kamerakopf angeschlossen ist, so dass das Operationsfeld auf einem Monitor betrachtet oder die erfassten Bilddaten einer Bildverarbeitung bereitgestellt werden können. Ein solches Endoskop geht beispielsweise aus EP 0 501 088 A1 hervor.

In optischen Abbildungssystemen treten unvermeidbar Abbildungsfehler auf, beispielsweise chromatische Aberration. Sie entsteht durch die Dispersion optischer Gläser, da Licht unterschiedlicher Wellenlänge unterschiedlich stark abgelenkt wird. Der Brechungsindex eines Glases ist keine konstante, sondern eine wellenlängenabhängige Funktion. Die chromatischen Aberrationen werden reduziert, indem Linsen mit unterschiedlicher Dispersion verwendet werden. So wird bei einem Achromat die axiale chromatische Aberration für zwei Wellenlängen korrigiert. Bei einem Apochromat wird die axiale chromatische Aberration für drei Wellenlängen korrigiert.

Bei einer als Achromat oder Apochromat ausgeführten optischen Einheit werden Gläser mit chromaler bzw. besonders geringer Dispersion eingesetzt. Diese Gläser werden als ED-Glas für "extra low dispersion glass", als SLD-Glas für "special low dispersion glass", als ELD-Glas für "extraordinary low dispersion glass" oder auch als UL-Glas für "ultra low dispersion glass" bezeichnet. Im Kontext der vorliegenden Beschreibung sollen solche Gläser allgemein als "ED-Glas" bezeichnet werden.

Chirurgische Instrumente, beispielsweise Endoskope, werden nach ihrer Benutzung aufbereitet. Hierzu werden sie mit einer Spül- und Reinigungslösung behandelt und vielfach in einem Desinfektionsschritt einer Wärmebehandlung in einem Autoklaven unterzogen.

Aus der US 5,569,163 A1 ist ein Einweg-Endoskop bekannt, dessen Okular eine optische Einheit aus zwei Linsen umfasst. Die Linsen sind aus einem Polymermaterial hergestellt. Die Okularfassung, in der die optische Einheit aufgenommen ist, ist aus einem verformbaren Polymer hergestellt, welches wasserlöslich ist. Wird das Endoskop gereinigt, so löst sich die Okularfassung vollständig auf.

Aus der US 2001/0036019 A1 ist ein Okular, beispielsweise für ein Teleskop, ein Binokular oder ein Mikroskop, bekannt, welches zur Korrektur der chromatischen Aberration eine verkittete Linse einsetzt.

Es ist eine Aufgabe der vorliegenden Erfindung, eine Okulareinrichtung für ein chirurgisches Instrument, ein Okular umfassend eine Okulareinrichtung sowie ein chirurgisches Instrument mit einem Okular anzugeben, wobei eine Temperaturfestigkeit der Okulareinrichtung, des Okulars und des chirurgischen Instruments, vor allem bei Temperaturen wie sie typischerweise beim Autoklavieren auftreten, verbessert werden soll.

Die Aufgabe wird gelöst durch eine Okulareinrichtung für ein chirurgisches Instrument, insbesondere für ein Endoskop, wobei die Okulareinrichtung eine Okularfassung und zumindest eine optische Einheit, die in der Okularfassung aufgenommen ist, umfasst, wobei die optische Einheit zumindest ein erstes optisches Element und ein damit verbundenes zweites optisches Element umfasst, wobei die Okulareinrichtung dadurch fortgebildet ist, dass das zweite optische Element aus einem zweiten Material mit anomaler Dispersion hergestellt ist und das erste und das zweite optische Element gemeinsam eine bezüglich chromatischer Aberration korrigierte optische Einheit bilden, wobei die Okularfassung einen Ausdehnungsraum für das zweite optische Element umfasst, wobei der Ausdehnungsraum einen für die optische Einheit vorgesehenen Bauraum in einer radialen Richtung erweitert.

Die erfindungsgemäße technische Lehre beruht auf der folgenden Erkenntnis: Die optischen Elemente einer bezüglich chromatischer Aberration korrigierten optischen Einheit, beispielsweise eines Achromaten oder eines Apochromaten, weisen neben unterschiedlichen Dispersionseigenschaften auch unterschiedliche thermische Ausdehnungskoeffizienten auf. Die zur Korrektur chromatischer Aberrationen verwendeten Materialien, beispielsweise Gläser mit anomaler Dispersion, dehnen sich bei Erwärmung stärker aus als Gläser mit normaler Dispersion. Außerdem sind Materialien mit anomaler Dispersion vielfach deutlich spröder als konventionelle Gläser.

Während die vorhandenen Unterschiede der thermischen Ausdehnungskoeffizienten bei vielen Anwendungen, beispielsweise bei Objektiven für die Fotografie, unkritisch sind, kommt ihnen beim Einsatz optischer Einheiten in einem chirurgischen Instrument eine nicht zu vernachlässigende Bedeutung zu. Bei der Aufbereitung des chirurgischen Instruments wird dieses in einem Autoklaven Temperaturen von über 100° C ausgesetzt. Bei dieser gegenüber Raumtemperatur deutlich erhöhten Temperatur tritt eine nennenswert unterschiedliche Ausdehnung der optischen Elemente der optischen Einheit auf. Das optische Element mit anomaler Dispersion zeigt eine größere thermische Ausdehnung als das optische Element mit normaler Dispersion.

Um einer Beschädigung der optischen Einheit, insbesondere des zweiten optischen Elementes, vorzubeugen, ist erfindungsgemäß vorgesehen, dass die Okularfassung einen Ausdehnungsraum für das zweite optische Element umfasst, wobei der Ausdehnungsraum einen für die optische Einheit vorgesehenen Bauraum in einer radialen Richtung erweitert . Gemäß einer vorteilhaften Weiterbildung ist außerdem in der Okularfassung lediglich das erste optische Element mechanisch aufgenommen. Mit anderen Worten ist also zum Aufnehmen der optischen Einheit in der Okularfassung zumindest ein mechanisches Verbindungselement zwischen der Okularfassung und dem ersten optischen Element vorhanden.

Die infolge der Wärmebehandlung und der daraus resultierenden Materialausdehnung auftretenden Kräfte treten somit zwischen dem ersten optischen Element und der Okularfassung auf. Das zweite optische Element, welches vielfach aus einem mechanisch spröderen Material hergestellt ist, wird keiner oder einer deutlich geringeren mechanischen Belastung ausgesetzt. Im Ergebnis wird die Temperaturfestigkeit der Okulareinrichtung deutlich verbessert, vor allem im Hinblick auf die bei einer Autoklavierbehandlung auftretenden Temperaturen.

Insbesondere ist vorgesehen, dass ausschließlich ein oder mehrere mechanische Verbindungselemente zwischen der Okularfassung und dem ersten optischen Element vorhanden sind. Mit anderen Worten ist keine direkte mechanische Verbindung zwischen dem zweiten optischen Element und der Okularfassung vorhanden.

Durch den vorgesehenen Ausdehnungsraum, der den für die optische Einheit vorgesehenen Bauraum in einer radialen Richtung erweitert, wird außerdem vorteilhaft ein mechanischer Kontakt zwischen dem zweiten optischen Element und der Okularfassung vermieden. Dieses dehnt sich während der Temperaturbehandlung in den vorhandenen Ausdehnungsraum hinein aus. Diese Maßnahme verbessert die Temperaturfestigkeit der Okulareinrichtung.

Ferner ist die Okulareinrichtung insbesondere dadurch fortgebildet, dass der Ausdehnungsraum einen Durchmesser der Okularfassung im Bereich des zweiten optischen Elements gegenüber einem Durchmesser der Okularfassung im Bereich des ersten optischen Elements vergrößert, wobei als Ausdehnungsraum insbesondere eine in die Okularfassung eingelassene, ferner insbesondere in Umfangsrichtung des zweiten Elements geschlossene, Nut oder Ausnehmung vorhanden ist. Die Nut oder Ausnehmung hat beispielsweise die Form eines Hohlzylinders, wobei auf dessen Innenseite der von dem zweiten optischen Element eingenommene Bauraum und auf dessen Außenseite die Innenseite der Okularfassung angrenzt.

Im Kontext der vorliegenden Beschreibung wird unter einem Bereich eines optischen Elementes ein bevorzugt zylinderförmiger Bereich verstanden, in dem sich das optische Element in Radialrichtung und in Längsrichtung, also entlang der optischen Achse, erstreckt. Mit anderen Worten ist der Durchmesser der Okularfassung auf der gesamten Länge des zweiten optischen Elements gegenüber den daran angrenzenden Bereichen erweitert. So wird vorteilhaft ein mechanischer Kontakt zwischen dem zweiten optischen Element und einer Innenseite der Okularfassung vermieden.

Ein mechanischer Kontakt zwischen dem zweiten optischen Element und einer Innenseite der Okularfassung kann ferner vermieden werden, indem die Okulareinrichtung bevorzugt dadurch fortgebildet ist, dass das zweite optische Element einen geringeren Durchmesser als das erste optische Element aufweist. Dies gilt insbesondere, wenn es sich bei den optischen Elementen um Linsen handelt.

Gemäß einer weiteren vorteilhaften Ausführungsform ist ferner vorgesehen, dass das mechanische Verbindungselement, mit der das erste optische Element in der Okularfassung gehalten ist, eine mit Klebstoff gefüllte Klebebohrung ist. Insbesondere sind mehrere mit Klebstoff gefüllte Klebebohrungen in der Okularfassung vorgesehen, durch die das erste optische Element entlang seines Umfangs in der Okularfassung gehalten ist.

Gemäß einer weiteren vorteilhaften Ausführungsform ist die Okulareinrichtung dadurch fortgebildet, dass das erste Material einen ersten thermischen Ausdehnungskoeffizienten und das zweite Material einen zweiten thermischen Ausdehnungskoeffizienten aufweist, wobei der zweite thermische Ausdehnungskoeffizient, insbesondere zumindest um einen Faktor zwei, größer ist als der erste thermische Ausdehnungskoeffizient.

Die Okulareinrichtung ist ferner vorteilhaft dadurch weitergebildet, dass das erste optische Element aus einem ersten Material mit einer ersten Abbe-Zahl und das zweite optische Element aus einem zweiten Material mit einer zweiten Abbe-Zahl hergestellt ist, wobei die zweite Abbe-Zahl größer als die erste Abbe-Zahl ist.

Die Abbé-Zahl V wird im Kontext der vorliegenden Beschreibung aus den wellenlängenabhängigen Brechungsindizes n des verwendeten Materials wie folgt berechnet:

$$V = \frac{n_e - 1}{n_{F'} - n_{C'}}$$

Die Abbe-Zahl ist ein Maß für die Dispersion des Materials, wobei eine niedrige Abbe-Zahl für eine hohe Dispersion und eine hohe Abbe-Zahl für eine niedrige Dispersion steht. In der oben genannten Formel stehen die Indizes e, F' und C' für die Fraunhofer-Linien e (Quecksilber, Wellenlänge 546,074 nm), F' (Cadmium bei 479,9915 nm) und C' (Cadmium bei 643,8469 nm).

Ferner ist insbesondere vorgesehen, dass das zweite Glas ein ED-Glas ist. Im Kontext der vorliegenden Beschreibung werden auch Gläser, die als SLD-Glas, ELD-Glas oder als UL-Glas bezeichnet werden als ED-Glas angesehen. Das ED-Glas hat insbesondere eine Abbe-Zahl größer als 75. Beispielsweise liegt die Abbe-Zahl des zweiten Materials zwischen 77 und 95, ferner insbesondere zwischen 77 und 80. Das als zweites Material verwendete Glas ist ferner insbesondere ein Fluoridglas, beispielsweise ein Calciumfluoridglas oder auch ein Fluorphosphatglas. Die zuvor genannten Materialparameter haben sich als vorteilhaft für die Herstellung der optischen Elemente der bezüglich chromatischer Aberration korrigierten optischen Einheit herausgestellt.

Handelt es sich bei den optischen Elementen um Linsen, so ist die optische Einheit ferner bevorzugt ein Achromat oder ein Apochromat. Ferner bevorzugt ist das zweite optische Element eine Okularlinse. Mit anderen Worten handelt es sich um eine außenliegende Linse der Okulareinrichtung. Diese wird ferner bevorzugt in einem chirurgischen Instrument verwendet.

Die Aufgabe wird ebenfalls gelöst durch ein Okular umfassend eine Okulareinrichtung nach einem oder mehreren der genannten Ausführungsformen, wobei das Okular dadurch fortgebildet ist, dass die optische Einheit eine Okularlinse des Okulars umfasst. Ferner wird die Aufgabe gelöst durch ein chirurgisches Instrument, insbesondere ein Endoskop, umfassend ein solches Okular.

Für das Okular bzw. das chirurgische Instrument mit einem solchen Okular treffen gleiche oder ähnliche Vorteile zu, wie sie bereits im Hinblick auf die Okulareinrichtung erwähnt wurden. Auf eine erneute Vorstellung wird daher verzichtet.

Das erste und das zweite optische Element, bei dem es sich bevorzugt um Linsen handelt, sind ferner bevorzugt miteinander verkittet oder verklebt. Es handelt sich bei den Linsen beispielsweise um bikonvexe, bikonkave oder konkavkonvexe Linsen. Die einander zugewandten Oberflächen des ersten und des zweiten optischen Elements sind bevorzugt formkomplementär ausgebildet. Neben verklebten oder verkitteten Linsensystemen sind auch Systeme mit Luftspalt vorgesehen. Dieser befindet sich zwischen dem ersten und zweiten optischen Element.

Die optischen Elemente sind gemeinsam mit ihrer Okularfassung bevorzugt in einem proximalen Bereich eines Endoskops angeordnet. Das Endoskop ist ferner bevorzugt ein Endoskop mit einem starren Endoskopschaft.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1    eine schematische und vereinfachte Seitenansicht eines chirurgischen Instruments, beispielhaft eines Endoskops,

Fig. 2    eine schematisch vereinfachte Längsschnittansicht durch eine Okulareinrichtung und

Fig. 3    einen weiteren schematisch vereinfachten Längsschnitt.

In den Zeichnungen sind jeweils gleiche oder gleichartige Elemente und/oder Teile mit denselben Bezugsziffern versehen, so dass von einer erneuten Vorstellung jeweils abgesehen wird.

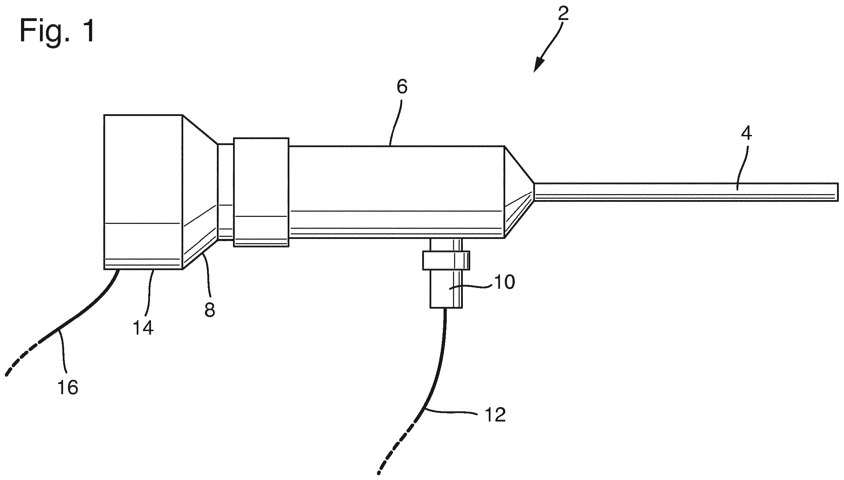

Fig. 1 zeigt in schematischer und vereinfachter Seitenansicht ein chirurgisches Instrument 2, beispielhaft ein Endoskop. An seinem distalen Ende umfasst dieses einen rohrförmigen Schaft 4 mit einer Optik, welches erlaubt, einen Operations- und Untersuchungsbereich, der distal vor einem freien Ende des Schafts 4 liegt, zu beobachten. Der Schaft 4 mündet in ein Gehäuse 6, das am proximalen Ende einen Okulartrichter 8 aufweist. Das Gehäuse 6 dient der Handhabung des chirurgischen Instruments 2. Seitlich an dem Gehäuse 6 befindet sich eine Lichtquelle 10, beispielsweise eine LED-Lichtquelle. Diese ist über ein Anschlusskabel 12 mit einer geeigneten Stromversorgung verbunden.

Am Okulartrichter 8 ist, schematisch dargestellt, ein Kamerakopf 14 mit einem nicht dargestellten Okularadapter angeordnet. Der Kamerakopf 14 erfasst das aus dem nicht dargestellten Okular des chirurgischen Instruments 2 austretende Licht mit einer geeigneten Optik und bildet dieses auf einen optischen Flächensensor ab, beispielsweise einem CCD- oder CMOS-Chip. Mittels eines Anschlusses 16 wird der Kamerakopf 14 mit Strom versorgt. Ferner ist es über den Anschluss 16 möglich, Bildsignale von dem Flächensensor des Kamerakopfs 14 an eine externe Auswerteeinheit zu übermitteln und Steuersignale an den Kamerakopf 14 zu übertragen.

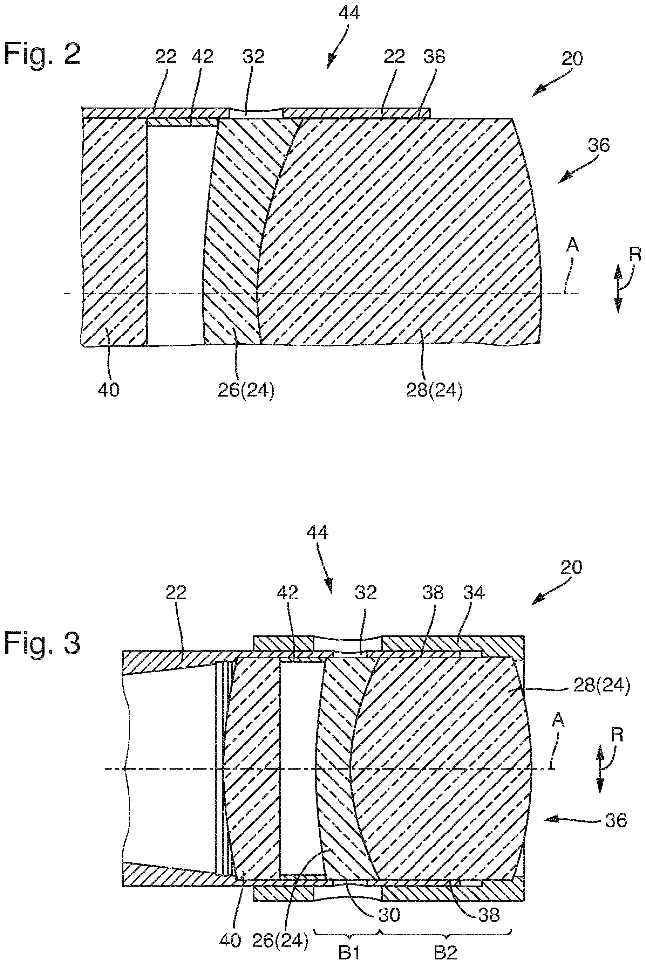

Fig. 2 zeigt in einem schematischen und vereinfachten Längsschnitt einen Teil einer Okulareinrichtung 20, wie sie im Bereich des Okulartrichters 8 am proximalen Ende des chirurgischen Instruments 2, beispielsweise eines Endoskops, vorgesehen ist (vgl. Fig. 1).

Die Okulareinrichtung 20 umfasst eine Okularfassung 22, in der eine optische Einheit 24 aufgenommen ist. Diese umfasst wiederum ein erstes optisches Element 26 und ein zweites optisches Element 28. Im dargestellten Ausführungsbeispiel handelt es sich bei den optischen Elementen 26, 28 beispielhaft um Linsen. Die beiden optischen Elemente 26, 28 bilden gemeinsam die optische Einheit 24, welche bezüglich chromatischer Aberration korrigiert ist. Ebenfalls beispielhaft ist ein Achromat dargestellt. Gemäß weiteren Ausführungsbeispielen umfasst die optische Einheit 24 weitere optische Elemente, so dass beispielsweise ein Apochromat bereitgestellt wird.

Das erste optische Element 26 ist aus einem ersten Material, beispielsweise einem ersten Glas oder einer ersten Glassorte, hergestellt. Das zweite optische Element 28 ist aus einem zweiten Material, beispielsweise aus einem zweiten Glas oder einer zweiten Glassorte, hergestellt. Das zweite Material, also beispielsweise die zweite Glassorte, ist ein Material mit anomaler Dispersion. Beispielsweise handelt es sich um ein ED-Glas. Im Kontext der vorliegenden Beschreibung werden als ED-Glas auch Gläser angesehen, die beispielsweise als SLD-Glas, ELD-Glas oder UL-Glas bezeichnet werden. Eine Abbe-Zahl des zur Herstellung des zweiten optischen Elements 28 verwendeten Glases ist insbesondere größer als 75. Beispielsweise wird zur Herstellung des zweiten optischen Elements 28 ein Fluorid-Glas verwendet.

Das zur Herstellung des ersten optischen Elements 26 verwendete erste Material, beispielsweise die erste Glassorte, weist eine erste Abbe-Zahl auf, während das zweite Material, aus dem das zweite optische Element 28 hergestellt ist, eine zweite Abbe-Zahl aufweist. Die zweite Abbe-Zahl ist bevorzugt größer als die erste Abbe-Zahl.

Die beiden optischen Elemente 26, 28, beispielsweise eine als erstes optisches Element 26 verwendete konkav-konvexe Linse und eine als zweites optisches Element 28 eingesetzte bikonvexe Linse sind bevorzugt miteinander verklebt oder verkittet. Die beiden optischen Elemente 26, 28 sind auf diese Weise miteinander verbunden. Es ist ebenso vorgesehen, dass zwischen den einander zugewandten Oberflächen des ersten und zweiten optischen Elements 26, 28, welche im dargestellten Ausführungsbeispiel miteinander verkittet oder verklebt sind, ein Luftspalt vorgesehen ist. Bei einer solchen Ausführungsform sind die beiden optischen Elemente 26, 28 auf andere Weise miteinander verbunden, beispielsweise durch ein Hüllrohr.

Die optische Einheit wird in die Okularfassung 22 von rechts nach links eingesetzt. Da nur eine relativ kurze Führungslänge vorhanden ist, ist eine Montagehilfe 34 vorgesehen. Fig. 3 zeigt in einer Detailansicht die Okulareinrichtung 20, ebenfalls in einer vereinfachten und schematischen Längsschnittansicht mit aufgesetzter Montagehilfe 34.

Die optische Einheit 24 ist in der Okularfassung 22 durch zumindest ein mechanisches Verbindungselement 30 aufgenommen. Beispielhaft handelt es sich bei diesem mechanischen Verbindungselement 30 um eine mit Klebstoff gefüllte Klebebohrung 32. Das mechanische Verbindungselement 32 greift lediglich am ersten optischen Element 26 an. Mit anderen Worten ist also das zweite optische Element 28 nicht direkt, sondern lediglich indirekt über das erste optische Element 26 mit der Okularfassung 22 verbunden.

Zur Verdeutlichung ist in Fig. 3 auf der oben dargestellten Seite der Okulareinrichtung 20 eine nicht mit Klebstoff gefüllte Klebebohrung 32 dargestellt. Die an der unteren Seite der Okulareinrichtung 20 dargestellte Klebebohrung 32 ist hingegen mit einem Klebstoff gefüllt, der in Kreuzschraffur dargestellt ist. Er bildet im ausgehärteten Zustand das mechanische Verbindungselement aus. Die Klebebohrung 32 erstreckt sich durch die Okularfassung 22 hindurch. Sie erstreckt sich zumindest näherungsweise in einer Radialrichtung R senkrecht zu einer optischen Achse A der optischen Einheit 24 durch die Wandung der Okularfassung 20 hindurch.

Die dargestellte Montagehilfe 34, welche die Okularfassung 22 an ihrer Außenseite bereichsweise umschließt, ist mit einer Freibohrung versehen, die über der Klebebohrung 32 angeordnet wird, so dass eine Klebedüse bis direkt an die Klebebohrung 32 verbracht werden kann. Die Montagehilfe 34 erstreckt sich an einer Okularaustrittsseite 36 über das zweite optische Element 28 hinweg. Die optische Einheit 24 wird so während der Montage, bis der Klebstoff ausgehärtet ist, sowohl in einer Längsrichtung, also parallel zur optischen Achse A, als auch in Radialrichtung R gehalten.

Von der Okularaustrittsseite 36 her wird die Okulareinrichtung 20 betrachtet. Sofern diese in einem Endoskop vorhanden ist, wird ein am distalen Ende des Endoskopschafts 4 vorhandenes Untersuchungsfeld aus dieser Richtung betrachtet (Fig. 1). Mit anderen Worten treten die distal in den Schaft 4 des chirurgischen Instruments 2 eintretenden Lichtstrahlen an der Okularaustrittsseite 36 aus dem chirurgischen Instrument 2 wieder aus und treffen beispielsweise auf einen flächigen Bildsensor des Kamerakopfs 14. Die bezüglich chromatischer Aberration korrigierte optische Einheit 24 umfasst, wie bereits erwähnt, ein erstes optisches Element 26 aus einem ersten Material und ein zweites optisches Element 28 aus einem zweiten Material. Das erste Material weist einen ersten thermischen Ausdehnungskoeffizienten und das zweite Material einen zweiten thermischen Ausdehnungskoeffizienten auf. Wird die beispielsweise in einem chirurgischen Instrument 2 vorhandene Okulareinrichtung 20 erwärmt, z.B. weil das chirurgische Instrument 2 in einem Autoklaven sterilisiert wird, so dehnen sich das erste optische Element 26 und das zweite optische Element 28 unterschiedlich stark aus. Diese durch den ersten bzw. zweiten Ausdehnungskoeffizienten bestimmte thermische Ausdehnung unterscheidet sich mitunter stark, da der zweite thermische Ausdehnungskoeffizient des zweiten optischen Elements 28 beispielsweise um einen Faktor zwei oder mehr größer ist als der erste thermische Ausdehnungskoeffizient des ersten optischen Elements 26. Um eine Beschädigung des zweiten optischen Elements 28 infolge seiner thermischen Ausdehnung, insbesondere in radialer Richtung R, also senkrecht zur optischen Achse A, vorzubeugen, ist ein Ausdehnungsraum 38 vorgesehen.

Der Ausdehnungsraum 38 für das zweite optische Element 28 erweitert den für die optische Einheit 24 in der Okularfassung 22 vorgesehenen Bauraum in Radialrichtung R. Aufgrund der annähernd maßstabsgetreuen Darstellung in den Fig. 2 und 3 ist der Ausdehnungsraum 38 in Radialrichtung R sehr klein und unterhalb der zur Darstellung verwendeten Strichbreite.

Der Ausdehnungsraum 38 erweitert einen Durchmesser der Okularfassung 22 in einem zweiten Bereich B2 des zweiten optischen Elements 28 gegenüber einem Durchmesser der Okularfassung 22 in einem ersten Bereich B1 des ersten optischen Elements 26. Der Ausdehnungsraum 38 hat beispielsweise die Form einer in die Okularfassung 22 eingelassenen Nut oder Ausnehmung. Diese Nut oder Ausnehmung ist insbesondere in Umfangsrichtung des zweiten optischen Elements 28 geschlossen.

Als Bereich B1, B2 des ersten bzw. zweiten optischen Elements 26, 28 werden bevorzugt diejenigen Bereiche verstanden, an denen das entsprechende optische Element 26, 28 mit seiner Außenkante an eine Innenseite der Okularfassung 22 anschlägt (im Fall des ersten optischen Elements 26) oder dieser zugewandt ist (im Fall des zweiten optischen Elements 28). Der Ausdehnungsraum 38 hat beispielsweise die Form eines Hohlzylinders. An eine Innenseite dieses Hohlzylinders grenzt eine Außenseite bzw. Mantelfläche des zweiten optischen Elements 28 an. An eine Außenseite bzw. äußere Mantelfläche dieses Hohlzylinders grenzt eine Innenseite bzw. innere Mantelfläche der Okularfassung 22 an. Der Ausdehnungsraum 38 bzw. die Nut oder Ausnehmung sind insbesondere in Umfangsrichtung des zweiten optischen Elements 28 geschlossen, so dass sich dieses gleichmäßig in alle Radialrichtungen R ausdehnen kann.

Alternativ oder zusätzlich zu einer in die Okularfassung 22 eingelassene Nut oder Ausnehmung ist ferner vorgesehen, dass das zweite optische Element 28 einen geringeren Durchmesser als das erste optische Element 26 aufweist. Auch dieser Durchmesser wird in Radialrichtung R betrachtet. Auf diese Weise wird ebenfalls ein Ausdehnungsraum 38 entlang der äußeren Mantelfläche des zweiten optischen Elements 28 geschaffen.

In der Okularfassung 22 befindet sich ferner eine weitere Linse 40 (vgl. Fig. 3), die über einen Distanzring 42 von der optischen Einheit 24 beabstandet und auf diese Weise auch in der Okularfassung 22 gehalten ist. Die optische Einheit 24 bildet gemeinsam mit der weiteren Linse 40 ein Okular 44, wie es beispielsweise von dem chirurgischen Instrument 2 (Fig. 1) in seinem Okulartrichter 8 umfasst ist. Dabei ist das zweite optische Element 28, welches aus einem Material mit anomaler Dispersion, beispielsweise einem ED-Glas, hergestellt ist, die Okularlinse dieses Okulars 44.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

Bezugszeichenliste

| | |
|---|---|
| 2 | chirurgisches Instrument |
| 4 | Schaft |
| 6 | Gehäuse |
| 8 | Okulartrichter |
| 10 | Lichtquelle |
| 12 | Anschlusskabel |
| 14 | Kamerakopf |
| 16 | Anschluss |
| 20 | Okulareinrichtung |
| 22 | Okularfassung |
| 24 | optische Einheit |
| 26 | erstes optisches Element |
| 28 | zweites optisches Element |
| 30 | mechanisches Verbindungselement |
| 32 | Klebebohrung |
| 34 | Montagehilfe |
| 36 | Okularaustrittsseite |
| 38 | Ausdehnungsraum |
| 40 | weitere Linse |
| 42 | Distanzring |
| 44 | Okular |
| | |
| A | optische Achse |
| R | Radialrichtung |
| B1 | Bereich des ersten optischen Elements |
| B2 | Bereich des zweiten optischen Elements |

**Patentansprüche**

1. Okulareinrichtung (20) für ein chirurgisches Instrument (2), insbesondere für ein Endoskop, wobei die Okulareinrichtung (20) eine Okularfassung (22) und zumindest eine optische Einheit (24), die in der Okularfassung (22) aufgenommen ist, umfasst, wobei die optische Einheit (24) zumindest ein erstes optisches Element (26) und ein damit verbundenes zweites optisches Element (28) umfasst, **dadurch gekennzeichnet, dass** das zweite optische Element (28) aus einem zweiten Material mit anomaler Dispersion hergestellt ist und das erste und das zweite optische Element (26, 28) gemeinsam eine bezüglich chromatischer Aberration korrigierte optische Einheit (24) bilden, wobei die Okularfassung (22) einen Ausdehnungsraum (38) für das zweite optische Element (28) umfasst, wobei der Ausdehnungsraum (38) einen für die optische Einheit (24) vorgesehenen Bauraum in einer radialen Richtung (R) erweitert.

2. Okulareinrichtung (20) nach Anspruch 1, **dadurch gekennzeichnet, dass** zum Aufnehmen der optischen Einheit (24) in der Okularfassung (22) zumindest ein mechanisches Verbindungselement (30) zwischen der Okularfassung (22) und dem ersten optischen Element (26) vorhanden ist.

3. Okulareinrichtung (20) nach Anspruch 1 oder 2, **dadurch gekennzeichnet, dass** der Ausdehnungsraum (38) einen Durchmesser der Okularfassung (22) im Bereich (B2) des zweiten optischen Elements (28) gegenüber einem Durchmesser der Okularfassung (22) im Bereich (B1) des ersten optischen Elements (26) vergrößert, wobei als Ausdehnungsraum (38) insbesondere eine in die Okularfassung (22) eingelassene, ferner insbesondere in Umfangsrichtung des zweiten optischen Elements (28) geschlossene, Nut oder Ausnehmung vorhanden ist.

4. Okulareinrichtung (20) nach einem der Ansprüche 1 bis 3, **dadurch gekennzeichnet, dass** das zweite optische Element (28) einen geringeren Durchmesser als das erste optische Element (26) aufweist.

5. Okulareinrichtung (20) nach Anspruch 2, **dadurch gekennzeichnet, dass** das mechanische Verbindungselement (30), mit der das erste optische Element (26) in der Okularfassung (22) gehalten ist, eine mit Klebstoff gefüllte Klebebohrung ist.

6. Okulareinrichtung (20) nach einem der Ansprüche 1 bis 5, **dadurch gekennzeichnet, dass** das erste Material einen ersten thermischen Ausdehnungskoeffizienten und das zweite Material einen zweiten thermischen Ausdehnungskoeffizienten aufweist, wobei der zweite thermische Ausdehnungskoeffizient, insbesondere zumindest um einen Faktor zwei, größer als der erste thermische Ausdehnungskoeffizient ist.

7. Okulareinrichtung (20) nach einem der Ansprüche 1 bis 6, **dadurch gekennzeichnet, dass** das erste optische Element (26) aus einem ersten Material mit einer ersten Abbe-Zahl und das zweite optische Element (28) aus einem zweiten Material mit einer zweiten Abbe-Zahl hergestellt ist, wobei die zweite Abbe-Zahl größer als die erste Abbe-Zahl ist.

8. Okulareinrichtung (20) nach einem der Ansprüche 1 bis 7, **dadurch gekennzeichnet, dass** das zweite Material ein ED-Glas ist und insbesondere eine Abbe-Zahl des zweiten Glases größer als 75 ist.

9. Okulareinrichtung (20) nach einem der Ansprüche 1 bis 8, **dadurch gekennzeichnet, dass** die optischen Elemente (26, 28) Linsen sind und die optische Einheit (24) ein Achromat oder ein Apochromat ist.

10. Okular (44) umfassend eine Okulareinrichtung (20) nach einem der Ansprüche 1 bis 9, **dadurch gekennzeichnet, dass** die optische Einheit (24) eine Okularlinse des Okulars (44) umfasst.

11. Chirurgisches Instrument (2), insbesondere Endoskop, umfassend ein Okular (44) nach Anspruch 10.

## Claims

1. An eyepiece device (20) for a surgical instrument (2), in particular for an endoscope, wherein the eyepiece device (20) comprises an eyepiece frame (22) and at least one optical unit (24) which is accommodated in the eyepiece frame (22), wherein the optical unit (24) comprises at least one first optical element (26) and a second optical element (28) connected therewith, **characterized in that** the second optical element (28) is manufactured from a second material with anomalous dispersion and the first and the second optical elements (26, 28) together form an optical unit (24) which is corrected with respect of chromatic aberration, wherein the eyepiece frame (22) comprises an expansion chamber (38) for the second optical element (28), wherein the expansion chamber (38) extends an installation space provided for the optical unit (24) in a radial direction (R).

2. The eyepiece device (20) according to Claim 1, **characterized in that** there is at least one mechanical connection element (30) between the eyepiece frame (22) and the first optical element (26) for accommodating the optical unit (24) in the eyepiece frame (22).

3. The eyepiece device (20) according to Claim 1 or 2, **characterized in that** the expansion chamber (38) increases a diameter of the eyepiece frame (22) in the region (B2) of the second optical element (28) with respect to a diameter of the eyepiece frame (22) in the region (B1) of the first optical element (26), wherein as the expansion chamber (38) in particular a groove or a recess is recessed into the eyepiece frame (22), wherein the groove or the recess is further in particular closed in a circumferential direction of the second optical element (28).

4. The eyepiece device (20) according to any one of Claims 1 to 3, **characterized in that** the second optical element (28) has a smaller diameter than the first optical element (26).

5. The eyepiece device (20) according to Claim 2, **characterized in that** the mechanical connection element (30), with which the first optical element (26) is held in the eyepiece frame (22), is a bonding bore filled with adhesive.

6. The eyepiece device (20) according to any one of Claims 1 to 5, **characterized in that** the first material has a first thermal expansion coefficient and the second material has a second thermal expansion coefficient, wherein the second thermal expansion coefficient is greater, in particular at least by a factor of two, than the first thermal expansion coefficient.

7. The eyepiece device (20) according to any one of Claims 1 to 6, **characterized in that** the first optical element (26) is manufactured from a first material with a first Abbe number and the second optical element (28) is manufactured from a second material with a second Abbe number, wherein the second Abbe number is greater than the first Abbe number.

8. The eyepiece device (20) according to any one of Claims 1 to 7, **characterized in that** the second material is an ED glass and an Abbe number of the second glass is in particular greater than 75.

9. The eyepiece device (20) according to any one of Claims 1 to 8, **characterized in that** the optical elements (26, 28) are lenses and the optical unit (24) is an achromat or an apochromat.

10. An eyepiece (44) comprising an eyepiece device (20) according to any one of Claims 1 to 9, **characterized in that** the optical unit (24) comprises an eyepiece lens of the eyepiece (44).

11. A surgical instrument (2), in particular an endoscope, comprising an eyepiece (44) according to Claim 10.

## Revendications

1. Dispositif oculaire (20) pour un instrument chirurgical (2), en particulier pour un endoscope, le dispositif oculaire (20) comprenant une monture d'oculaire (22) et au moins une unité optique (24) logée dans la monture d'oculaire (22), l'unité optique (24) comprenant au moins un premier élément optique (26) et un deuxième élément optique (28) qui lui est associé, **caractérisé en ce que** le deuxième élément optique (28) est fabriqué en un deuxième matériau à dispersion anormale et **en ce que** les premier et deuxième éléments optiques (26, 28) forment ensemble une unité optique (24) corrigée en aberration chromatique, la monture d'oculaire (22) comprenant un espace d'expansion (38) pour le deuxième élément optique (23), l'espace d'expansion (38) élargissant un espace de montage prévu pour l'unité optique (24) dans une direction radiale (R)

2. Dispositif oculaire (20) selon la revendication 1, **caractérisé en ce que**, pour recevoir l'unité optique (24) dans la monture d'oculaire (22), il est prévu au moins un élément de liaison mécanique (30) entre la monture d'oculaire (22) et le premier élément optique (26).

3. Dispositif oculaire (20) selon la revendication 1 ou la revendication 2, **caractérisé en ce que** l'espace d'expansion (38) présente un diamètre agrandi de la monture d'oculaire (22) dans la zone (B2) du deuxième élément optique (28) par rapport à un diamètre de la monture d'oculaire (22) dans la zone (B1) du premier élément optique (26), l'espace d'expansion (38) étant en particulier une rainure ou un évidement ménagé dans la monture d'oculaire (22), qui est en outre notamment fermé dans le sens circonférentiel du deuxième élément optique (28).

4. Dispositif oculaire (20) selon l'une des revendications 1 à 3, **caractérisé en ce que** le deuxième élément optique (28) a un diamètre inférieur à celui du premier élément optique (26).

5. Dispositif oculaire (20) selon la revendication 2, **caractérisé en ce que** l'élément de liaison mécanique (30), avec lequel le premier élément optique (26) est maintenu dans la monture d'oculaire (22), est un alésage d'adhésion rempli d'adhésif.

6. Dispositif oculaire (20) selon l'une des revendications 1 à 5, **caractérisé en ce que** le premier matériau a un premier coefficient de dilatation thermique et le deuxième matériau a un deuxième coefficient de dilatation thermique, le deuxième coefficient de dilatation thermique étant supérieur au premier coefficient de dilatation thermique, en particulier d'au moins un facteur deux.

7. Dispositif oculaire (20) selon l'une quelconque des revendications 1 à 6, **caractérisé en ce que** le premier élément optique (26) est constitué en un premier matériau ayant un premier nombre d'Abbe et le deuxième élément optique (28) est constitué en un deuxième matériau ayant un deuxième nombre d'Abbe, le deuxième nombre d'Abbe étant supérieur au premier nombre d'Abbe.

8. Dispositif oculaire (20) selon l'une des revendications 1 à 7, **caractérisé en ce que** le deuxième matériau est un verre ED, et un nombre d'Abbe du deuxième verre est en particulier supérieur à 75.

9. Dispositif oculaire (20) selon l'une des revendications 1 à 8, **caractérisé en ce que** les éléments optiques (26, 28) sont des lentilles et l'unité optique (24) est un achromat ou un apochromat.

10. Oculaire (44) comprenant un dispositif oculaire (20) selon l'une des revendications 1 à 9, **caractérisé en ce que** l'unité optique (24) comprend une lentille oculaire de l'oculaire (44).

11. Instrument chirurgical (2), en particulier endoscope, comprenant un oculaire (44) selon la revendication 10.

EP 3 261 511 B1

Fig. 1

Fig. 2

Fig. 3

**EP 3 261 511 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0501088 A1
- US 5569163 A1
- US 20010036019 A1